# EUROPEAN PATENT APPLICATION

(11) **EP 1 967 182 A1**
(43) Date of publication of application: **10.09.2008**
(21) Application number: 07004715.4
(22) Date of filing: 07.03.2007
(51) Int. Cl.: A61K 9/20, A61K 9/28, A61K 31/427

(54) **Pharmaceutical composition comprising a salt of rosigliatazone**

(71) Applicant: KRKA, tovarna zdravil, d.d., Novo mesto, 8501 Novo mesto (SI)
(72) Inventor: German, Tamara, 8000 Novo mesto (SI); Vrbinc, Miha, 8000 Novo mesto (SI); Zupancic, Silvo, SLO-8000 Novo mesto (SI); Stefanic, Marko, 8340 Crnomelj (SI)
(74) Representative: UEXKÜLL & STOLBERG

(57) **Abstract**

The present invention relates to a pharmaceutical composition comprising
(a) 0.1 - 10 % by weight of a salt of rosiglitazone as active ingredient, and
(b) 0.2 - 4.0 % by weight of disintegrant
and a process for producing the same.

## Description

The present invention relates to a novel solid pharmaceutical composition comprising a salt of rosiglitazone and a process for its preparation.

### Background of the invention

Rosiglitazone is known to have hypoglycaemic and hypolipidaemic activity and has been disclosed in EP 0 306 228. Rosiglitazone is the INN name for 5-[4-[2-(N-methyl-N-(2-pyridyl)amino)ethoxy]benzyl]-thiazolidine-2,4-dione, which is represented by the formula:

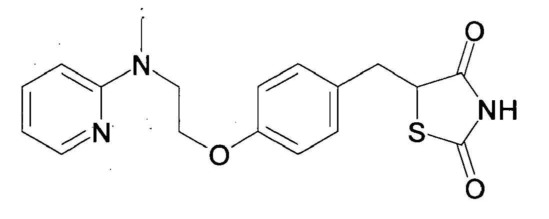

Rosiglitazone free base is practically insoluble in water. WO 94/05659 discloses certain salts of rosiglitazone, particularly rosiglitazone maleate, which are said to be significantly more soluble in water than the corresponding free base. In addition these salts are said to show good aqueous stability and good stability in the solid form.

WO 98/55122 describes granular compositions comprising rosiglitazone maleate in a concentrated form and a pharmaceutically acceptable carrier, which are adapted to be diluted so as to provide a composition for administration. The concentrated compositions preferably contain from 5 to 20 % by weight of rosiglitazone in combination with a disintegrant, a binder and a diluent. A suitable disintegrant is sodium starch glycollate which is used in an amount of 5 % by weight. The concentrate is admixed with pharmaceutically acceptable excipients to provide the final dosage form comprising about 5 % by weight of sodium starch glycollate. The final compositions comprise 2 to 12 mg of the active ingredient and are said to be useful for the treatment of diabetes mellitus.

WO 03/050113 and WO 03/050114 disclose a process for preparing rosiglitazone hydrogensulfate salt and rosiglitazone sulfate salt, respectively. These salts are said to be alternative salt forms of rosiglitazone which have properties suitable for pharmaceutical processing on a commercial scale, especially in manufacturing processes which require or generate heat.

It was found that salts of rosiglitazone generally have low solubility and that the release of the active ingredient from pharmaceutical compositions is therefore insufficient.

### Description of the invention

It is the object of the present invention to provide a pharmaceutical composition comprising a salt of rosiglitazone as the active ingredient which is highly stable during storage and dissolves quickly upon contact with water.

It has surprisingly been found by the present inventors that this object can be achieved by using a pharmaceutical composition comprising
(a) 0.1 - 10 % by weight of a salt of rosiglitazone as active ingredient, and
(b) 0.2 - 4.0 % by weight of disintegrant.

Unless indicated otherwise, all percentages given herein are by weight based on the total weight of the composition.

The active ingredient (a) in the composition is preferably a sulfuric acid addition salt of rosiglitazone. Examples of sulfuric acid addition salts of rosiglitazone are rosiglitazone sulfate, i.e. a 2:1 salt of rosiglitazone in protonated form and the SO₄²⁻ anion; rosiglitazone hydrogensulfate, i.e. a 1:1 salt of rosiglitazone in protonated form and the HSO₄⁻ anion; a mixed (1:1) salt of rosiglitazone comprising rosiglitazone in protonated form, a further cation such as an alkali, alkaline earth or ammonium cation, and the SO₄²⁻ anion; and mixtures thereof. More preferably, the sulfuric acid addition salt of rosiglitazone is selected from rosiglitazone sulfate, rosiglitazone hydrogensulfate and mixtures thereof.

According to the present invention, an active ingredient (a) can be used in any polymorphic or pseudopolymorphic form, e.g. in the form of a solvate or hydrate or in amorphous form.

It is particularly preferred that the active ingredient (a) is rosiglitazone sulfate having an X-ray powder diffraction pattern exhibiting peaks at the following diffraction angles as obtained using a Phillips PW3040/60 X'Pert PRO diffractometer with CuKα radiation of 1,541874 Å:

| No. | Diffraction angles [°; 2Θ] | d-spacing [Å] | Rel. Int. [%] |
|---|---|---|---|
| 1 | 13.2 | 6.69 | 14 |
| 2. | 13.6 | 6.51 | 15 |
| 3 | 14.9 | 5.94 | 14 |
| 4 | 17.5 | 5.08 | 37 |
| 5 | 18.1 | 4.90 | 60 |
| 6 | 18.7 | 4.75 | 55 |
| 7 | 18.9 | 4.69 | 26 |
| 8 | 21.0 | 4.23 | 100 |
| 9 | 22.9 | 3.88 | 40 |
| 10 | 25.0 | 3.56 | 32 |
| 11 | 26.2 | 3.40 | 32 |
| 12 | 27.4 | 3.26 | 18 |
| 13 | 28.4 | 3.14 | 10 |
| 14 | 29.0 | 3.08 | 14 |
| 15 | 30.1 | 2.97 | 11 |

It is further preferred that the composition comprises 0.3 - 7 %, more preferably 0.5 - 5 % by weight of active ingredient (a), preferably of a sulfuric acid addition salt of rosiglitazone.

The composition of the present invention also comprises at least one pharmaceutically acceptable disintegrant (component (b)). The disintegrant is preferably selected from crospovidone, pregelatinised starch, sodium starch glycollate, carboxymethylcellulose sodium (CMC-Na), carboxymethylcellulose calcium (CMC-Ca), cross-linked CMC-Na, polacrilin potassium (a potassium salt of a copolymer of methacrylic acid and divinyl benzene), low substituted hydroxypropylcellulose (i.e. hydroxypropylcellulose having between 5.0% and 16.0% of hydroxypropoxy groups -OCH₂CHOHCH₃), and mixtures thereof. More preferably the disintegrant is selected from sodium starch glycollate, cross-linked CMC-Na, crospovidone, carboxymethylcellulose calcium (CMC-Ca) and low-substituted hydroxypropylcellulose, and mixtures thereof.

It is further preferred that the composition comprises 0.5 - 4 %, more preferably 1 - 4.0 % by weight of disintegrant (b).

It was surprisingly found by the present inventors that the dissolution rate of compositions comprising a rosiglitazone salt as the active ingredient can be improved by adjusting the amount of disintegrant to be within the above ranges, i.e. by reducing the amount of disintegrant used in compositions known in the state of the art, rather than increasing it. Although better than the solubility of rosiglitazone free base, the solubility of rosiglitazone salts such as rosiglitazone sulfate and rosiglitazone hydrogensulfate is still poor and it was totally unexpected that pharmaceutical compositions that dissolve more quickly upon contact with water can be obtained by using an amount of disintegrant that is lower instead of higher than that commonly used.

The composition may advantageously also comprise further pharmaceutically acceptable excipients. Further pharmaceutically acceptable excipients are preferably selected from the group of diluents, surfactants (surface active agents), antioxidants, chelating agents, disintegrants, binders, lubricants, glidiants.

Examples of suitable diluents include microcrystalline cellulose, powdered cellulose, lactose (anhydrous and monohydrate), compressible sugar, fructose, dextrates, sugar alcohols such as mannitol, sorbitol, maltitol, xylitol, lactitol, or other sugars such as saccharose, raffinose, trehalose, or fructose, siliconised microcrystalline cellulose, calcium hydrogenphosphate, calcium carbonate, calcium lactate, and mixtures thereof. Preferably, the diluent is selected from microcrystalline cellulose, lactose, mannitol, and mixtures thereof.

It is further preferred that the composition comprises 1 - 99 %, more preferably 10 - 95 %, even more preferably 30 - 93 %, most preferably 70 - 90 % by weight of diluent.

Preferred binders are polyvinylpyrrolidone, hydroxyethylcellulose, hydroxypropylcellulose, hydroxypropylmethylcellulose or other cellulose ethers, starch, pre-gelatinised starch, polymethacrylate, and mixtures thereof. It is further preferred that the binder is partially soluble in water. Examples of binders with partial solubility in water include polyvinylpyrrolidones and hydroxypropylmethylcellulose.

It is further preferred that the composition comprises 0.1 - 10 %, more preferably 0.3 - 7%, even more preferably 0.5 - 5% by weight of binder.

Examples for suitable lubricants include stearic acid or stearic acid salts, such as magnesium stearate, magnesium palmitate, magnesium oleate, hydrogenated vegetable oil, hydrogenated castor oil, talc, sodium stearyl fumarate, macrogols or mixtures thereof. Preferred lubricants are stearic acid, magnesium stearate, hydrogenated vegetable oil, hydrogenated castor oil and talc.

It is further preferred that the composition comprises 0.1 - 10 %, more preferably 0.1 - 7 %, even more preferably 0.1 - 5 % by weight of lubricant.

Surfactant molecules may be classified based on the nature of the hydrophilic group within the molecule. The four main groups of surfactants are defined as follows:
1) Anionic surfactants, wherein the hydrophilic group carries a negative charge, such as carbonyl (RCOO⁻), sulfonate (RSO₃⁻) or sulfate (ROSO₃⁻)· Pharmaceutically acceptable examples include potassium laurate, CH₃(CH₂)₁₀COO⁻K⁺, and sodium lauryl sulfate, CH₃(CH₂)₁₁SO₄⁻Na⁺
2) Cationic surfactants, wherein the hydrophilic group carries a positive charge (e.g., quaternary ammonium halides, R₄N⁺Cl⁻). Pharmaceutically acceptable examples include cetrimide, a mixture consisting mainly of tetradecyl (ca. 68 %), dodecyl (ca. 22%), and hexadecyltrimethylammonium bromides (ca. 7 %), as well as benzalkonium chloride, a mixture of alkylbenzyldimethylammonium chlorides of the general formula [C₆H₅CH₂N⁺(CH₃)₂R]Cl⁻, wherein R represents a mixture of the alkyls from C₈H₁₇ to C₁₈H₃₇.
3) Ampholytic surfactants (also called zwitterionic surfactants), wherein the molecule contains, or can potentially contain, both a negative and a positive charge, (e.g., the sulfobetaines, RN⁺(CH₃)₂CH₂CH₂SO₃⁻). Pharmaceutically acceptable examples include N-Dodecyl-N,N-dimethylbetaine, C₁₂H₂₅N⁺(CH₃)₂CH₂COO⁻ .
4) Nonionic surfactants, wherein the hydrophile carries no charge but derives its water solubility from highly polar groups such as hydroxyl or polyoxyethylene (OCH₂CH₂O-) groups. Pharmaceutically acceptable examples include polyoxyethylated glycol monoethers (e.g. cetomacrogol), sorbitan esters (Spans®) and polysorbates (Tweens®).

Special examples of surfactants are poloxamers that are defined as nonionic polyoxyethylene-polyoxypropylene copolymers. They are chemically similar in composition, differing only in the relative amounts of propylene and ethylene oxides added during manufacture.

Lubricants, glidants and antiadherents used in the compositions of the present invention, are herein not defined as surfactants.

It is further preferred that the composition comprises 0.1 - 5 %, more preferably 0.1 - 4 %, even more preferably 0.1 - 3 % by weight of surfactant.

According to a preferred embodiment of the present invention, the composition does not comprise surfactant. It has been found that solubility and dissolution rate of the composition can be enhanced by omitting a surfactant.

As a further component, the composition of the present invention preferably contain an acidic agent. It has been found that solubility of the active ingredient and dissolution of the compositions according to the invention can be enhanced by the addition of an acidic agent. An acidic agent can be selected from the group of pharmaceutically acceptable inorganic or organic acids. Inorganic acids include e.g. hydrochloric, sulfonic, phosphonic acid etc. Organic acid may be selected form any one of organic carboxylic acid, preferably an aliphatic organic carboxylic acid having 2 to 20 carbon atoms, for example, tartaric acid, malic acid, succinic acid, glutaric acid, glutamic acid, maleic acid, mandelic acid, citric acid, alginic acid, asparaginic acid, stearic acid, lactic acid, acetic acid, tosylic acid, besylic acid (benzenemonosulfonic acid), salicylic acid, benzoic acid and the like.

It is further preferred that the composition comprises 0.1 - 5 %, more preferably 0.1 - 4 %, even more preferably 0 - 3 % by weight of acidic agent.

Preferably, the active ingredient (a) is in the form of particles having a d₁₀ value of 1-20µm, more preferably 3 - 15 µm, a d₅₀ value of 5 - 50 µm, more preferably 10 - 45 µm, or a d₉₀ value of 20 - 150 µm, more preferably 25 - 110 µm. The dₓ value indicates that a certain percentage X by volume of the particles has a size below a certain limit. For example, a d₉₀ value of 150 µm means that 90 % by volume of the particles have a diameter below 150 µm.

In addition to the active ingredient (a), the particles of active ingredient (a) may contain further additives, but it is preferred that they essentially and more preferably completely consist of the active ingredient (a).

In a preferred embodiment, the composition according to the invention comprises a granulate containing one or more excipients. More preferably, the granulate has a d₉₀ value of at least 125 µm, still more preferably at least 250 µm.

In a composition according to the invention comprising a granulate, the composition can comprise active ingredient (a) that is contained in the granulate and/or active ingredient (a) that is not contained in the granulate.

Where the composition comprises a granulate, any excipient can generally be comprised either in the intragranular or the extragranular phase or both in the intragranular and the extragranular phase. For instance, having a disintegrant comprised in the extragranular phase provides for disintegration of tablet cores into granules while having a disintegrant comprised in the intragranular phase provides for disintegration of individual granules into primary particles. Thus, having a disintegrant comprised in the intragranular and the extragranular phase will allow for fast and complete release of the active ingredient from the mixture with excipients.

The composition according to the invention is preferably in the form of a coated or uncoated tablet, e.g. a fast disintegrating tablet or orally disintegrating tablet, a pill, a capsule, e.g. a soft or hard gelatine capsule, pellets, suppositories, lozenges, sacchets, etc. The composition can also take the form of a powder mixture, of a granulate or of mini tablets filled in capsules. The dosage form is preferably suitable for oral application. An immediate release composition is especially preferred.

The compositions are preferably formulated in a unit dosage form, each dosage form containing about 1 to 100 mg, more preferably 1 to about 16 mg of rosiglitazone in the form of a sulfuric acid addition salt. The term "unit dosage form" refers to physically discrete units suitable as unitary dosages for human objects and other mammals, each unit containing a predetermined quantity of rosiglitazone calculated to produce the desired therapeutic effect, in association with a suitable pharmaceutical excipient as listed above.

Tablets can be coated with conventional materials used for film coating, e.g. as described in Pharmaceutical Coating Technology (G. Cole (ed.), 1995). Film coating formulations usually contain polymer(s), plasticizer(s), colorant (s) /opacifier (s), and vehicle(s). Also, minor quantities of flavors, surfactants and waxes may be present in the coating.

Polymers preferably used in film coating are either cellulose derivatives, such as cellulose ethers, or acrylic polymers and copolymers. Other examples include high molecular weight polyethylene glycols, polyvinyl pyrrolidone, polyvinyl alcohol and waxy materials. Their function usually is to prevent bad mouth feel and/or taste and in some cases degradation, e.g. oxidation of active ingredient and/or excipients used.

Cellulose ethers typically used in film coating are hydroxyethylcellulose, hydroxypropylcellulose, hydroxypropylmethylcellulose and methylcellulose. Acrylic polymers represent a group of synthetic polymers with diverse functionalities. They can be modified to enhance swelling and permeability, e.g. by the incorporation of other substances such as water soluble cellulose ethers and starches in order to ensure complete disintegration/dissolution of the film.

Examples for typical plasticizers used in film coating are polyols, such as glycerol, propylene glycol, macrogols; organic esters, such as phthalate esters, dibutyl sebacetate, citrate esters, triacetin; and oils/glycerides, such as castor oil, acetylated monoglycerides, and fractionated coconut oil.

Colourants/opacifiers include organic dyes and their lakes, inorganic colors, and natural colors.

Different materials from each group can also be combined in defined ratios. Film coating suspensions can also be used as ready-to-make preparations which are available on the market, e.g. Opadry (manufactured by Colorcon) etc.

Film coating dispersion can be prepared by using different solvents (water, alcohols, ketones, esters, chlorinated hydrocarbons), preferably water.

A coating composition which comprises (calculated on dry material)
1-99%, preferably 1-95% by weight of polymer,
1-50%, preferably 1-40% by weight of plasticizer, and
0.1-20%, preferably 0.1-10% by weight of colorant/opacifier
is particularly preferred.

Preferably, the amount of coating applied per tablet is 0.5 to 10 % by weight, more preferably 1 to 7 % by weight based on solid weight of non-coated tablet.

It was surprisingly found that the compositions according to the invention overcome the problems associated with conventional rosiglitazone formulations. Particularly, the composition dissolves quickly upon contact with water.

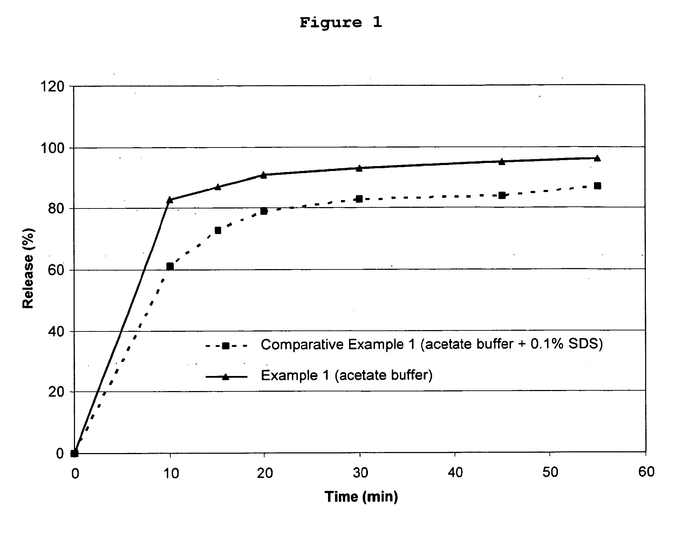

Figure 1 shows a graph illustrating the dissolution profile of two different tablet formulations. The tablet formulation of Example 1 contains an amount of disintegrant in accordance with the present invention while the tablet formulation of Comparative Example 1 contains a higher amount.

The percentage of released active ingredient is plotted against the time (minutes). The dissolution rate of the tablets according to Example 1 was determined in acetate buffer of pH 4.5 according to USP definition. The tablets of Comparative Example 1 exhibited only a very slow and incomplete release of active ingredient in acetate buffer. Therefore, 0.1% sodium dodecyl sulfate (SDS) was added in order to improve the dissolution of the tablets. Nevertheless the tablets according to the present invention still showed a quicker and more complete release of the active agent.

The pharmaceutical composition according to the invention can be prepared by a granulation process, such as dry or wet granulation, or by a direct compression process.

The active ingredient is first prepared according to any suitable synthetic process and then purified, e.g. by crystallization or other means. Rosiglitazone hydrogensulfate is preferably prepared by the process described in WO03/050113, rosiglitazone sulfate by the process described in WO 03/050114. Rosiglitazone hydrogensulfate is preferably used in crystal form as described in WO 03/050113 or in amorphous form.

The active ingredient is preferably converted to particles as defined above. Particle size is controlled by crystallization or milling of active ingredient. With milling, particles of less then 3 µm average diameter may be produced. Examples of suitable milling equipment include air jet mills, ball mills or hammer.

Next, the active ingredient is mixed or diluted with an excipient or a mixture of excipients. The one or more excipients are preferably converted to a granulate, e.g. by wet or dry granulation, before mixing with the active ingredient. More preferably, the granulate has a d₉₀ value of at least 125 µm, still more preferably at least 250 µm.

Mixing of the excipients or of excipients with the active ingredient may be effected in conventional devices used for mixing of powders, e.g. motionless (passive) mixers, fluidized bed, diffusion, biconic diffusion, uniconic, biconic, turbular, cubic, planetary, Y-, V-shaped or high-shear mixers.

The mixture of the active ingredient with the one or more excipients can then be converted to the final dosage form. For instance, tablets can be obtained by compressing the mixture to tablets. Alternatively, the mixture can be filled e.g. into capsules or sacchets. The mixture can also be subjected to granulation such as dry granulation or wet granulation and the obtained granulate is then compressed to tablets, filled e.g. into capsules or sacchets.

Compression of the mixture to tablets, can be effected using an automatic rotary compressing machine as available from different manufacturers of equipment for use in pharmaceutical industry.

The tablets can be provided with a coating. Preferably, the tablets are film coated, for instance by use of a fluid bed apparatus (e.g. Wurster coating system) or conventional coating pans for use in pharmaceutical industry.

Preferably, the composition is prepared by a process comprising the steps of
(1) preparing a granulate of one or more excipients,
(2) mixing the granules of step (1) with the active ingredient (a) and one or more excipients,
(3) optionally compressing the obtained mixture to tablets, and
(4) optionally providing the tablets with a coating.

It is also possible to add active ingredient in granulation step (1) in order to obtain a granulate which additionally comprises active ingredient. This granulate is mixed with further active ingredient (a) and one or more excipients in step (2). This process results in the formation of tablets which contain active ingredient in granulate phase and in the extragranular phase of the tablets.

According to an alternative embodiment, the composition is prepared by a process comprising the steps of
(1') preparing a granulate of active ingredient (a) and one or more excipients,
(2') optionally mixing the granules of step (1) with further excipients,
(3') optionally compressing the obtained mixture to tablets, and
(4') optionally providing the tablets with a coating.

It has been found that a composition according to the invention which is prepared by wet granulation is stable at ICH stability testing conditions, as defined in ICH guideline Q1A(R2): Stability Testing of New Drug Substances and Products. Surprisingly, a composition according to the invention is even stable at such conditions if active ingredient is contained in the granulate.

For drying of granulate obtained by wet granulation, conventional drying devices such as a fluid-bed dryer or drying chambers (non-vacuumized or vacuumized) can be used. The granulate is preferably dried by a fluid bed process. It .was found that drying of the granulate in a fluid-bed dryer results in the formation of a product having a well-reproducible composition and well-reproducible processing properties.

The wetting of a mixture of excipients or a mixture of active ingredient and excipient(s) can be performed by spraying water, alcohol, water/alcohol mixture or aqueous, alcoholic or aqueous/alcoholic granulating liquid onto the mixture. Wetting can be achieved by conventional pharmaceutical techniques in conventional granulation equipment. Wetting can also be affected by direct addition of water, alcohol, water/alcohol mixture or aqueous, alcoholic or aqueous/alcoholic granulating liquid to a mixture of excipients or a mixture of active ingredient and excipient(s) during mixing in a mixing device, e.g. high-shear mixer. The term "aqueous, alcoholic or aqueous/alcoholic granulating liquid" refers to an aqueous, alcoholic or aqueous/alcoholic dispersion which contains purified water or demineralised water and/or lower alcohols (methanol, ethanol, isopropanol) as diluents and a solid substance which is dispersed, suspended or dissolved in the diluent. The dispersed substance can be an excipient used in the present compositions, e.g. binder, surfactant or acidic agent.

The present invention will further be illustrated by means of the following examples.

### Preparative examples

### Preparative example 1

2 g (5.6 mmol) of rosiglitazone base was suspended in 25 ml of 2-butanone and the mixture was heated to the reflux temperature. To the formed solution, 0.6 ml (10 mmol) of 96 % H₂SO₄ were added, stirred at this temperature for 15 min and then cooled to room temperature. The precipitate formed was filtered and dried at room temperature overnight. 2.4 g (94 %) of rosiglitazone sulfate was isolated.
T: 186-190 °C
IR: 1755, 1696, 1646, 1617, 1514, 1315, 1222, 1166, 1071, 864, 773, 581
Elemental Analysis for C₁₈H₁₉N₃O₃S * H₂SO₄: C 47.36 (47.46); H 4.70 (4.65); N 9.01 (9.22)

### Preparative example 2

2.3 g (6.5 mmol) of rosiglitazone base was added to 29 ml of 4-methyl-2-pentanone and the mixture was heated to the reflux temperature. To the formed solution, 0.7 ml (12 mmol) of 96 % H₂SO₄ were added, stirred at this temperature for 15 min and then cooled to room temperature. The precipitate formed was filtered and dried at room temperature overnight. 2.7 g (92%) of rosiglitazone sulfate was isolated.
T: 190-192 °C
IR: 1755, 1696, 1646, 1617, 1514, 1315, 1222, 1166, 1071, 864, 773, 581
Elemental Analysis for C₁₈H₁₉N₃O₃S * H₂SO₄: C 47.34 (47.46); H 4.65 (4.65); N 8.97 (9.22)

### Preparative example 3

1.36 g (3.8 mmol) of rosiglitazone base was suspended in 17 ml of tetrahydrofurane and the mixture was heated to the reflux temperature. To the formed solution, 0.4 ml (6.9 mmol) of 96 % H₂SO₄ were added, stirred at this temperature for 15 min and then cooled to room temperature. The precipitate formed was filtered and dried at room temperature overnight. 1.7 g (98 %) of rosiglitazone sulfate was isolated.
T: 188-190 °C
IR: 1754, 1698, 1646, 1618, 1514, 1315, 1222, 1165, 1071, 865, 772, 581
Elemental Analysis for C₁₈H₁₉N₃O₃S * H₂SO₄: C 47.14 (47.46); H 4.68 (4.65); N 8.97 (9.22)

### Preparative example 4

2.08 g (5.8 mmol) of rosiglitazone base was added to 20.ml of 1,4-dioxane and the mixture was heated to the reflux temperature. To the formed solution, 0.33 ml (5.8 mmol) of 96.% H₂SO₄ were added, stirred at this temperature for 15 min and then cooled to room temperature. The precipitate formed was filtered and dried at room temperature overnight. 1.8 g (69%) of rosiglitazone sulfate was isolated.
T: 198-203 °C
IR: 1754, 1696, 1646, 1618, 1513, 1314, 1221, 1165, 1071, 865, 772, 580
Elemental Analysis for C₁₈H₁₉N₃O₃S * H₂SO₄: C 47.33 (47.46) ; H 4.61 (4.65); N 9.09 (9.22)

### Preparative example 5

6 g (16.8 mmol) of rosiglitazone base was added to 240 ml of acetone. The mixture is heated to the reflux temperature and then the obtained solution was cooled to 30 to 40°C. After that a solution of H₂SO₄ (0.96 ml, 17.3 mmol) in acetone (40 ml) was slowly added and the mixture was cooled to room temperature. The suspension was stirred at this temperature for 1 h. The product was separated, washed with fresh acetone and dried at room temperature overnight. 7.7 g (92 %) of rosiglitazone sulfate was isolated.
T: 178-18.4 °C
IR: 1754, 1696, 1646, 1618, 1519, 1314, 1222, 1166, 1070, 864, 712, 580, 532, 521
Elemental Analysis for C₁₈H₁₉N₃O₃S * H2SO4: C 47. 67 (47.46); H 4.75 (4.65); N 9.08 (9.22)

### Examples

### Comparative Example 1.

30.45 g of rosiglitazone sulfate, 9 g of asparaginic acid, 12 g of sodium starch glycolate, 12 g of hydroxypropylmethylcellulose, 48 g of microcrystalline cellulose and 1.98 g of lactose monohydrate were homogenized in high-shear mixer.

85 g of purified water was sprayed onto the mixture of rosiglitazone and excipients as listed above. The granulate obtained was dried in fluid-bed dryer at inlet air temperature 60°C. Loss on drying (measurement performed on apparatus Mettler Toledo HR73 halogen moisture analyzer at 85°C for 20 minutes at 85°C for 20 minutes) of the sieved granulate was 0.93% by weight.

33 g of sodium starch glycolate, 131 g of microcrystalline cellulose and 423 g of lactose monohydrate were added to the granulate obtained above and mixed in a high-shear mixer. Finally, 4.5 g of magnesium stearate was added to obtain compression mixture. Loss on drying (measurement performed on apparatus Mettler Toledo HR73 halogen moisture analyzer at 85°C for 20 minutes at 85°C for 20 minutes) of the compression mixture was 1.26% by weight.

The compression mixture was compressed into cores with theoretical weight of 300 mg. The disintegration time of the cores was 30 seconds (Ph.Eur., Purified Water, 37 °C) and the average hardness (Erweka tester) was 122N.

Cores were coated in an automatic coating pan with water-based film coating suspension of ready-to-make mixture, commercially available as Opadry. Theoretical weight of the film coated tablets was 309 mg.

A graph illustrating the dissolution profile of tablets is represented in Figure 1.

### Example 1

30.45 g of rosiglitazone sulfate, 9 g of asparaginic acid, 17 g of sodium starch glycolate, 12 g of hydroxypropylmethylcellulose, 48 g of microcrystalline cellulose and 210 g of lactose monohydrate were homogenized in high-shear mixer.
78 g of purified water was sprayed onto the mixture of rosiglitazone and excipients as listed above. The granulate obtained was dried in fluid-bed dryer at inlet air temperature 60°C. Loss on drying (measurement performed on apparatus Mettler Toledo HR73 halogen moisture analyzer at 85°C for 20 minutes at 85°C for 20 minutes) of the sieved granulate was 1.08% by weight.

6 g of sodium starch glycolate, 131 g of microcrystalline cellulose and 433 g of lactose monohydrate were added to the granulate obtained above and mixed in a high-shear mixer. Finally, 4.5 g of magnesium stearate was added to obtain compression mixture. Loss on drying (measurement performed on apparatus Mettler Toledo HR73 halogen moisture analyzer at 85°C for 20 minutes at 85°C for 20 minutes) of the compression mixture was 1.09% by weight. Flowability of the compression mixture was 12.9 seconds and angle of repose was 32.0° (both measurements were performed on apparatus Pharmatest PTG). Bulk volume was 1.90 ml/g and tapped volume 1.58 ml/g (measurements performed on apparatus Engelsmann).
Sieve analysis of the compression mixture was performed on apparatus Alpina 200 LS:

| **Sieve size (µm)** | **% passed** |
|---|---|
| 71 | 3.30 |
| 125 | 15.00 |
| 250 | 67.00 |
| 500 | 92.95 |
| 710 | 96.85 |
| 1250 | 100.00 |

The compression mixture was compressed into cores with theoretical weight of 300 mg. The disintegration time of the cores was 45 seconds (Ph.Eur., Purified Water, 37 °C) and the average hardness (Erweka tester) was 116N.

Cores were coated in an automatic coating pan with water-based film coating suspension of ready-to-make mixture, commercially available as Opadry. Theoretical weight of the film coated tablets was 309 mg.

A graph illustrating the dissolution profile of tablets is represented in Figure 1.

### Example 2

30.45 g of rosiglitazone sulfate, 9 g of asparaginic acid, 18 g of croscarmellose sodium, 12 g of hydroxypropylmethylcellulose, 48 g of microcrystalline cellulose and 210 g of lactose monohydrate were homogenized in high-shear mixer.
120 g of purified water was sprayed onto the mixture of rosiglitazone and excipients as listed above. The granulate obtained was dried in fluid-bed dryer at inlet air temperature 60°C. Loss on drying (measurement performed on apparatus Mettler Toledo HR73 halogen moisture analyzer at 85°C for 20 minutes at 85°C for 20 minutes) of the sieved granulate was 1.06% by weight.

9 g of croscarmellose sodium, 131 g of microcrystalline cellulose and 428 g of lactose monohydrate were added to the granulate obtained above and mixed in a high-shear mixer. Finally, 4.5 g of magnesium stearate was added to obtain compression mixture. Loss on drying (measurement performed on apparatus Mettler Toledo HR73 halogen moisture analyser at 85°C for 20 minutes at 85°C for 20 minutes) of the compression mixture was 1.08% by weight.

The compression mixture was compressed into cores with theoretical weight of 300 mg. The disintegration time of the cores was 35 seconds (Ph.Eur., Purified Water, 37 °C) and the average hardness (Erweka tester) was 120N.

Cores were coated in an automatic coating pan with water-based film coating suspension of ready-to-make mixture as described in Example 1.

### Example 3

30.45 g of rosiglitazone sulfate, 17 g of sodium starch glycolate, 12 g of hydroxypropylmethylcellulose, 48 g of microcrystalline cellulose and 216 g of lactose monohydrate were homogenized in high-shear mixer.

75 g of purified water was sprayed onto the mixture of rosiglitazone and excipients as listed above. The granulate obtained was dried in fluid-bed dryer at inlet air temperature 60°C. Loss on drying (measurement performed on apparatus Mettler Toledo HR73 halogen moisture analyzer at 85°C for 20 minutes at 85°C for 20 minutes) of the sieved granulate was 0.98% by weight.

6 g of sodium starch glycolate, 129 g of microcrystalline cellulose and 438 g of lactose monohydrate were added to the granulate obtained above and mixed in a high-shear mixer. Finally, 4.5 g of magnesium stearate was added to obtain compression mixture. Loss on drying (measurement performed on apparatus Mettler Toledo HR73 halogen moisture analyzer at 85°C for 20 minutes at 85°C for 20 minutes) of the compression mixture was 1.02% by weight. The compression mixture was compressed into cores with theoretical weight of 300 mg. The disintegration time of the cores was 40 seconds (Ph.Eur., Purified Water, 37°C) and the average hardness (Erweka tester) was 118N.

Cores were coated in an automatic coating pan with water-based film coating suspension of ready-to-make mixture as described in Example 1.

### Example 4

30.45 g of rosiglitazone sulfate, 18 g of croscarmellose sodium, 12 g of hydroxypropylmethylcellulose, 48 g of microcrystalline cellulose and 210 g of lactose monohydrate were homogenised in high-shear mixer.

115 g of purified water was sprayed onto the mixture of rosiglitazone and excipients as listed above. The granulate obtained was dried in fluid-bed dryer at inlet air temperature 60°C. Loss on drying (measurement performed on apparatus Mettler Toledo HR73 halogen moisture analyser at 85°C for 20 minutes at 85°C for 20 minutes) of the sieved granulate was 1.03% by weight.

9 g of croscarmellose sodium, 132 g of microcrystalline cellulose and 436 g of lactose monohydrate were added to the granulate obtained above and mixed in a high-shear mixer. Finally, 4.5 g of magnesium stearate was added to obtain compression mixture. Loss on drying (measurement performed on apparatus Mettler Toledo HR73 halogen moisture analyzer at 85°C for 20 minutes at 85°C for 20 minutes) of the compression mixture was 1.09% by weight.

The compression mixture was compressed into cores with theoretical weight of 300 mg. The disintegration time of the cores was 35 seconds (Ph.Eur., Purified Water, 37 °C) and the average hardness (Erweka tester) was 123N.

Cores were coated in an automatic coating pan with water-based film coating suspension of ready-to-make mixture as described in Example 1.

### Example 5

30.45 g of rosiglitazone sulfate, 17 g of sodium starch glycolate, 12 g of hydroxypropylmethylcellulose, 48 g of microcrystalline cellulose and 216 g of lactose monohydrate were homogenised in high-shear mixer.

86 g of purified water was sprayed onto the mixture of rosiglitazone and excipients as listed above. The granulate obtained was dried in fluid-bed dryer at inlet air temperature 60°C. Loss on drying (measurement performed on apparatus Mettler Toledo HR73 halogen moisture analyzer at 85°C for 20 minutes at 85°C for 20 minutes) of the sieved granulate was 1.05 by weight.

7 g sodium lauryl sulfate, 6 g of sodium starch glycolate, 129 g of microcrystalline cellulose and 430 g of lactose monohydrate were added to the granulate obtained above and mixed in a high-shear mixer. Finally, 4.5 g of magnesium stearate was added to obtain compression mixture. Loss on drying (measurement performed on apparatus Mettler Toledo HR73 halogen moisture analyzer at 85°C for 20 minutes at 85°C for 20 minutes) of the compression mixture was 1.11% by weight. The compression mixture was compressed into cores with theoretical weight of 300 mg. The disintegration time of the cores was 45 seconds (Ph.Eur., Purified Water, 37 °C) and the average hardness (Erweka tester) was 124N.

Cores were coated in an automatic coating pan with water-based film coating suspension of ready-to-make mixture as described in Example 1.

### Example 6

30.45 g of rosiglitazone sulfate, 17 g of sodium starch glycolate, 12 g of hydroxypropylmethylcellulose, 48 g of microcrystalline cellulose and 216 g of lactose monohydrate were homogenized in high-shear mixer.

73 g of purified water was sprayed onto the mixture of rosiglitazone and excipients as listed above. The granulate obtained was dried in fluid-bed dryer at inlet air temperature 60°C. Loss on drying (measurement performed on apparatus Mettler Toledo HR73 halogen moisture analyser at 85°C for 20 minutes at 85°C for 20 minutes) of the sieved granulate was 1.01 by weight.

7 g polysorbate 80, 6 g of sodium starch glycolate, 129 g of microcrystalline cellulose and 430 g of lactose monohydrate were added to the granulate obtained above and mixed in a high-shear mixer. Finally, 4.5 g of magnesium stearate was added to obtain compression mixture. Loss on drying (measurement performed on apparatus Mettler Toledo HR73 halogen moisture analyzer at 85°C for 20 minutes at 85°C for 20 minutes) of the compression mixture was 1.04% by weight. The compression mixture was compressed into cores with theoretical weight of 300.mg. The disintegration time of the cores was 40 seconds (Ph.Eur., Purified Water, 37 °C) and the average hardness (Erweka tester) was 113N.

Cores were coated in an automatic coating pan with water-based film coating suspension of ready-to-make mixture as described in Example 1.

## Claims

1. Pharmaceutical composition comprising
(a) 0.1 - 10 % by weight of a salt of rosiglitazone as active ingredient, and
(b) 0.2 - 4.0 % by weight of disintegrant.

2. The pharmaceutical composition of claim 1, wherein the active ingredient (a) is a sulfuric acid addition salt of rosiglitazone.

3. The pharmaceutical composition of claim 2, wherein the sulfuric acid addition salt of rosiglitazone is selected from rosiglitazone sulfate, rosiglitazone hydrogensulfate, and mixtures thereof.

4. The pharmaceutical composition of any one of the preceding claims, wherein the disintegrant (b) is selected from the group consisting of sodium starch glycollate, cross-linked carboxymethylcellulose sodium, crospovidone, carboxymethylcellulose calcium and low-substituted hydroxypropylcellulose.

5. The pharmaceutical composition of any one of the preceding claims, wherein the diluent is selected from the group consisting of microcrystalline cellulose, lactose and mannitol, or mixtures thereof.

6. The pharmaceutical composition of any one of the preceding claims, wherein the binder is selected from the group consisting of polyvinylpyrrolidone, hydroxypropylmethylcellulose.

7. The pharmaceutical composition of any one of the preceding claims which comprises 0.5 - 4 %, preferably 1 - 4.0 % by weight of disintegrant (b).

8. The pharmaceutical composition of any one of the preceding claims which further comprises 1 - 99 %, preferably 10 - 95 %, more preferably 30 - 93 %, most preferably 70 - 90 % by weight of diluent.

9. The pharmaceutical composition of any one of the preceding claims which further comprises 0.1 - 10 %, preferably 0.3-7 %, more preferably 0.5-5 % by weight of binder.

10. The pharmaceutical composition of any one of the preceding claims which further comprises 0.1 - 10, preferably 0.1 - 7 %, more preferably 0.1 - 5 % by weight of lubricant.

11. The pharmaceutical composition of any one of the preceding claims which further comprises 0.1 - 5 %, preferably 0.1 - 4 %, more preferably 0 - 3 % by weight of surfactant.

12. The pharmaceutical composition of any one of the preceding claims which further comprises 0.1 - 5 %, preferably 0.1 - 4%, more preferably 0 - 3 % by weight of acidic agent.

13. The pharmaceutical composition of any one of the preceding claims, wherein the active ingredient (a) is in the form of particles having a d₁₀ value of 1 - 20 µm, preferably 3- 15. µm.

14. The pharmaceutical composition of any one of the preceding claims, wherein the active ingredient (a) is in the form of particles having a d₅₀ value of 5 - 50 µm, preferably 10 - 45 µm.

15. The pharmaceutical composition of any one of the preceding claims, wherein the active ingredient (a) is in the form of particles having a d₉₀ value of 20 - 150 µm, preferably 25 - 110 µm.

16. The pharmaceutical composition of any one of the preceding claims, wherein the composition comprises a granulate containing one or more excipients.

17. The pharmaceutical composition of claim 16, wherein the granulate has a d₉₀ value of at least 125 µm, preferably at least 250 µm.

18. The pharmaceutical composition of claim 16 or 17 comprising active ingredient (a) that is contained in the granulate.

19. The pharmaceutical composition of any one of claims 16 to 18 comprising active ingredient (a) that is not contained in the granulate.

20. The pharmaceutical composition of any one of the preceding claims that is in the form of a tablet.

21. Process for the preparation of a composition according to any one of claims 1 to 20 comprising the steps of
(1) preparing a granulate of one or more excipients,
(2) mixing the granules of step (1) with the active ingredient (a) and one or more excipients.

22. Process for the preparation of a composition according to any one of claims 1 to 20 comprising the steps of
(1) preparing a granulate of active ingredient (a) and one or more excipients,
(2) mixing the granules of step (1) with the active ingredient (a) and one or more excipients.

23. Process for the preparation of a composition according to any one of claims 1 to 20 comprising the steps of
(1) preparing a granulate of active ingredient (a) and one or more excipients,
(2) mixing the granules of step (1) with one or more excipients.

24. The process of any one of claims 21 to 23 further comprising (3) compressing the obtained mixture to tablets.

25. The process of claim 24 further comprising (4) coating of the tablets of step (3).
